Europäisches Patentamt

European Patent Office

Office européen des brevets

(19)

(11) **EP 0 431 153 B1**

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(51) Int. Cl.$^6$: **A61B 3/125**, A61B 3/117, A61B 3/15

(21) Anmeldenummer: 90917757.8

(22) Anmeldetag: **19.06.1990**

(86) Internationale Anmeldenummer:
**PCT/DE90/00461**

(87) Internationale Veröffentlichungsnummer:
**WO 90/15570 (27.12.1990 Gazette 1990/29)**

(54) **Vorrichtung zur Untersuchung und/oder Behandlung des Auges**

Device for examining and/or treating the eye

Appareil pour l'examen et/ou traitement de l'oeil.

(84) Benannte Vertragsstaaten:
**CH DE FR GB IT LI**

(30) Priorität: **19.06.1989 DE 3919985**

(43) Veröffentlichungstag der Anmeldung:
**12.06.1991 Patentblatt 1991/24**

(73) Patentinhaber:
**G. RODENSTOCK INSTRUMENTE GMBH
85521 Ottobrunn (DE)**

(72) Erfinder:
• **REIS, Werner
  D-8000 München 50 (DE)**

• **PLESCH, Andreas
  D-8000 München 40 (DE)**
• **WILMS, Karl-Heinz
  D-8089 Emmering (DE)**

(74) Vertreter: **Münich, Wilhelm, Dr.
Kanzlei Münich, Steinmann, Schiller
Wilhelm-Mayr-Str. 11
80689 München (DE)**

(56) Entgegenhaltungen:
DE-A- 2 301 468        FR-A- 2 271 585
US-A- 4 213 478        US-A- 4 410 245
US-A- 4 598 984        US-A- 4 664 490
US-A- 4 728 183        US-A- 4 753 526

**Beschreibung**

**Technisches Gebiet**

Die Erfindung bezieht sich auf eine Vorrichtung zur Untersuchung und/oder zur Behandlung des Auges gemäß dem Oberbegriff des Patentanspruchs 1.

Eine Vorrichtung zur Untersuchung und/oder zur Behandlung des Auges mit den Merkmalen des Oberbegriffs des Anspruchs 1 ist beispielsweise aus der US-A-4 213 678 bekannt. Bei dieser bekannten Vorrichtung ist die optische Wirkung des mit zwei sphärischen Flächen versehenen Kontaktglases in die Berechnung des Strahlengangs des Untersuchungsgeräts mit einbezogen, bei dem sich ein Zwischenbild des den Augenhintergrund abtastenden Laserstrahls in einer Ebene vor dem Kontaktglas befindet. Das Kontaktglas ist damit unverzichtbarer Bestandteil des Strahlengangs des Untersuchungsgeräts, in diesem Falle eines Scanning-Laser-Ophthalmoskops.

Damit ist es nicht möglich, das Kontaktglas aus dem Strahlengang herauszunehmen, wenn es bei bestimmten Untersuchungs- und/oder Behandlungssituationen stört. Dies ist beispielsweise dann der Fall, wenn die Untersuchungsperson, die in der Regel das Kontaktglas mit einer Hand hält, für bestimmte Aufgaben beide Hände benötigt.

Weiterhin gibt es bestimmte Untersuchungs- und/oder Behandlungssituationen, bei denen ein möglichst großer "freier Arbeitsabstand", d.h. ein möglichst großer Abstand zwischen dem Scheitel des Auges und dem Scheitel der letzten Fläche des Augenuntersuchungsgerätes, d.h. der am nächsten zu dem untersuchten Auge gelegenen Fläche des Augenuntersuchungsgerätes erforderlich ist.

Es sind deshalb in letzter Zeit verstärkt Vorrichtungen zur Untersuchung und/oder zur Behandlung des Auges vorgeschlagen worden, deren Strahlengang so aufgebaut ist, daß auf die Verwendung eines Kontaktglases verzichtet wird. Nur beispielhaft wird hierzu auf die US-Patentschriften 4 764 005, 4 768 873 und 4 854 692 sowie die EP-A 0 145 563 verwiesen, in denen Scanning Laser Ophthalmoskope beschrieben sind, bei denen kein Kontaktglas verwendet wird.

Aus der US-A-4 598 984 ist ein Kontaktglas zur Untersuchung und/oder zur Behandlung des Augenhintergrundes mit einem Untersuchungsgerät bekannt, dessen optischer Aufbau "eigentlich" nur zur Untersuchung der vorderen Augenabschnitte erlaubt. Solche Untersuchungsgeräte sind beispielsweise Spaltlampengeräte, die vom optischen Aufbau des sogenannten Spaltlampen-Mikroskops zur Untersuchung der Hornhaut sowie der vorderen Augenabschnitte ausgelegt sind. Durch die Verwendung dieses Kontaktglases kann das Einsatzgebiet derartiger Geräte auch auf die Untersuchung und/oder Behandlung des Augenhintergrunds ausgedehnt werden. Das aus der US-A-4 589 984 bekannte Kontaktglas kann jedoch nicht in Verbindung mit Augenuntersuchungsgeräten verwendet werden, die zur Untersuchung und/oder zur Behandlung des Augenhintergrundes ausgelegt sind, da die "Addition" der Wirkung des Untersuchungsgeräts und des Kontaktglases eine optische Wirkung ergeben wurde, die ein Scharfeinstellen auf den Augenhintergrund unmöglich macht. Aus der US-A-4 728 183 ist eine Kontaktglasanordnung bekannt, die zwei Linsen mit optischer Wirkung aufweist, von denen eine, nämlich die auf die Cornea aufgesetzte Linse eine konkav/konvexe Linse ist, wobei die Flächen annähernd gleichen Krümmungsradius haben. Die andere Linse ist eine bikonvexe Linse mit vergleichsweise großer optischer Wirkung, so daß das Kontaktglas ebenfalls in die Berechnung des Strahlengangs des Augenuntersuchungsgeräts mit einbezogen werden muß.

Aus der DE-A-23 01 468 ist eine Kontaktglasanordnung mit beweglichen Spiegelflächen bekannt, die damit auch die Beobachtung der seitlichen Abschnitte des Auges erlaubt.

Hierfür dient auch die aus der US-A-4 664 490 bekannte Kontaktglasanordnung, die ebenfalls Spiegelflächen aufweist, die allerdings nicht beweglich sind.

Es hat sich nun herausgestellt, daß Augenärzte zur Laserbehandlung des Augenhintergrunds als Untersuchungs- bzw. Beobachtungsgeräte Spaltlampengeräte mit zusätzlichen Kontaktgläsern gegenüber Geräten bevorzugen, die eigentlich zur Untersuchung des Augenhintergrunds gedacht sind, und bei denen kein zusätzliches Kontaktglas verwendet wird.

Erfindungsgemäß ist nun erkannt worden, daß die Ursache hierfür sein dürfte, daß sich bei einer Laserbehandlung des Augenhintergrundes ohne Verwendung eines Kontaktglases eine Reihe von Problemen ergeben:

Diese werden beispielsweise durch den Lidschlag, die unvermeidlich auftretende Augenunruhe sowie dadurch hervorgerufen, daß bei längerem Aufhalten des Auges ohne Lidschlag, wie dies beispielweise beim Aufhalten durch eine Klammer der Fall ist, die Hornhaut austrocknet. Das Austrocknen der Hornhaut deformiert diese, so daß sich eine wesentliche Bildverschlechterung ergibt.

**Darstellung der Erfindung**

Der Erfindung liegt die Aufgabe zugrunde, die insbesondere bei der Laserbehandlung des Augenhintergrundes mit einem für den Augenhintergrund ausgelegten Augen-Untersuchungsgerät durch Lidschlag, Augenunruhe etc. auftretenden Probleme zu lösen.

Eine erfindungsgemäße Lösung dieser Aufgabe ist im Anspruch 1 angegeben. Weiterbildungen der Erfindung sind in den Patentansprüchen 2 folgende gekennzeichnet.

Erfindungsgemäß ist erkannt worden, daß sich die durch Lidschlag, Augenunruhe oder Aufhalten des Augenlides durch eine Klammer bei einem für den Augenhintergrund ausgelegten Untersuchungsgerät ergebenden Probleme dadurch lösen lassen, daß auch bei einem derartigen Gerät ein Kontaktglas verwendet wird. Erfindungsgemäß wird dieses Kontaktglas im Vergleich zu bekannten Kontaktgläsern jedoch so ausgebildet, daß die auf das Auge aufgesetzte Linse zumindest im Bereich der optischen Achse (praktisch) keine sphärische Wirkung hat. Hierunter wird insbesondere verstanden, daß für die sphärische Wirkung D im Bereich der optischen Achse der auf das Auge aufgesetzten Linse gilt:

$$- 0,5\,dpt < D < 0,5\,dpt.$$

Dabei ist - wenn überhaupt eine geringe sphärische Wirkung vorgesehen ist - diese bevorzugt negativ, um typische Abbildungsfehler des Untersuchungsgeräts zu kompensieren.

Durch das erfindungsgemäß verwendete Kontaktglas ohne sphärische Wirkung wird der Strahlengang des Augenuntersuchungsgeräts, also beispielsweise des Scanning-Laserophthalmoskops nicht verändert. Dennoch ist gewährleistet, daß kein Lidschlag auftreten kann, der die Beobachtung und/oder Laserbehandlung des Augenhintergrundes stören würde, und daß die Hornhaut durch das Aufhalten des Augenlides aufgrund der zwischen der augenseitigen Fläche der Linse und dem Auge befindlichen Flüssigkeit nicht austrocknet. Darüberhinaus wird die Augenunruhe durch das aufgesetzte Kontaktglas wesentlich reduziert.

Andererseits ist es jedoch möglich, das Augenuntersuchungsgerät weiterhin ohne Kontaktglas zu verwenden, so daß der Arzt je nach Untersuchungs- und/oder Behandlungssituation die Wahl zwischen einer Verwendung des Untersuchungsgeräts ohne und mit Kontaktglas hat.

Als Augenuntersuchungsgeräte können in Verbindung mit dem "Null-Kontaktglas" beliebige Untersuchungsgeräte verwendet werden, die eine Beobachtung des Augenhintergrundes ohne zusätzliches Kontaktglas gestatten. Derartige Untersuchungsgeräte sind beispielsweise herkömmliche Funduskameras, Ophthalmoskope oder sogenannte Scanning-Laserophthalmoskope, wie sie beispielsweise aus der EP-A-0 145 563, den japanischen Patentveröffentlichungen 61-5730 und 50-138822, der deutschen Patentschrift 32 45 939 oder der WO 88/03396 bekannt sind. Deshalb soll im folgenden auf die Ausbildung der Untersuchungsgerät nicht näher eingegangen werden.

Weiterbildungen der Erfindung sind in den Unteransprüchen angegeben.

Erfindungsgemäß ist erkannt worden, daß ein normales menschliches Auge chromatische Abweichungen für außeraxiale Strahlen aufweist, die die Abbildung insbesondere peripherer Bereiche des Auges deutlich verschlechtern. Deshalb ist es besonders bevorzugt, wenn die auf das Auge aufgesetzte Linse zwar im Bereich der optischen Achse praktisch keine sphärische Wirkung hat, aber außerhalb der optischen Achse eine "asphärische" Zusatzwirkung aufweist, die die chromatischen Abbildungsfehler des menschlichen Auges zumindest teilweise kompensiert. Dabei ist bevorzugt die asphärische Fläche die dem Auge abgewandte Fläche, also die Fläche, die nicht auf das Auge aufgesetzt wird, da bei dieser Fläche die Freiheitsgrade für die "Asphärizität" größer sind als bei der augenseitigen Fläche, deren Kontur im wesentlichen der Krümmung der Cornea folgen muß.

Da - wie weiterhin erfindungsgemäß erkannt worden ist - das menschliche Auge durch das sogenannte Gullstrand'sche Augenmodell nur unzureichend beschrieben wird, ist es bevorzugt, wenn die asphärische Fläche bei Annäherung der Flächenschnitte durch eine Ellipse einen Kegelschnittkoeffizienten K hat, für den gilt:

$$-0,65 < K < -0,55.$$

Ausdrücklich wird jedoch darauf hingewiesen, daß die asphärische Fläche in der Regel keine Flächenschnitte hat, die exakt Ellipsenform sind, sondern daß die Flächenschnitte von der Ellipsenform abweichen können.

In der Regel ist ferner die asphärische Fläche eine rotationssymmetrische Fläche. In besonderen Fällen ist es jedoch möglich, daß die asphärischen Abweichungen nicht rotationssymmetrisch sind. Ferner ist es auch möglich, eine torische oder atorische Fläche, d.h. eine Fläche mit Zylinderwirkung und nicht sphärischen Hauptschnitten im Falle von Astigmatismus-behafteten Augen zu verwenden.

Weiterhin ist wenigstens ein Spiegel vorgesehen, der die Beobachtung und/oder Behandlung peripherer Augenabschnitte ermöglicht. Durch die Verwendung eines derartigen - an sich bekannten - Spiegels wird gerade der Anwendungsbereich eines als Untersuchungsgerät verwendeten Scanning-Ophthalmoskops, das einen vergleichsweise kleinen vertikalen bzw. horizontalen Bildwinkel von typischerweise ca. 30° hat, wesentlich erweitert. Dieser Spiegel, der bevorzugt außerhalb des Beobachtungs- und/oder Behandlungsstrahlengangs für den zentralen Bereich angeordnet ist und damit die Beobachtung des zentralen Bereichs nicht stört, ermöglicht gerade bei einem Scanning-Laserophthalmoskop, das in der Regel lediglich eine Schwenkmöglichkeit um eine vertikale Achse, d.h eine Verstellmöglichkeit in einer annähernd horizontalen Ebene aufweist, nicht nur eine Vergrößerung des "horizontalen Bildwinkels", sondern bei einer entsprechenden Drehung des Kontaktglases auch eine Vergrößerung des "vertikalen Bildwinkels".

Darüberhinaus ist es möglich, den Umlenk-Spiegel beweglich auszuführen, so daß es möglich ist, den beobachte-

EP 0 431 153 B1

ten Fundusausschnitt kontinuierlich zu variieren.

Selbstverständlich ist es möglich, nicht nur einen Umlenkspiegel vorzusehen, sondern eine Reihe von Umlenkspiegeln, die nacheinander durch eine entsprechende Schwenkbewegung des Untersuchungs- bzw. Behandlungsgeräts und/oder eine entsprechende Drehung des Kontaktglases in den Strahlengang des Gerät überführt werden. Die verschiedenen Sätze von Spiegeln ermöglichen dann die Beobachtung und/oder Behandlung unterschiedlicher peripherer Bereiche. Besonders bevorzugt ist es, zwei Sätze von Spiegeln vorzusehen, die insbesondere um 90° gegeneinander verschwenkt sind, und die die Beobachtung und/oder Behandlung unterschiedlicher peripherer Bereiche ermöglichen. In jedem Falle ist es jedoch von Vorteil, wenn sich

der zentrale Beobachtungs- und Behandlungsbereich und der bzw. die über die Spiegel beobachtbaren Bereiche ohne Überschneidung aneinander anschließen. Beispielsweise bei einem Scanning-Laserophthalmoskop mit nachgeschalteter Bildverarbeitung können dann die einzelnen, auf unterschiedliche Weise beobachteten Bereiche "nahtlos" aneinander anschließend auf einem Monitor dargestellt werden.

Die verschiedenen Spiegel können selbstverständlich Planspiegel und/oder Prismen und damit Elemente sein, die den Strahlengang lediglich umlenken. Genausogut ist es aber auch möglich, daß wenigstens ein Teil der Spiegel eine optische Wirkung aufweist, die bevorzugt dazu dienen kann, daß Abbildungsfehler in den peripheren Augenbereichen wenigstens teilweise kompensiert werden oder eine Beobachtung des Kammerwinkels möglich wird.

Bei der einen weiteren Ausgestaltung sind die auf das Auge aufsetzbare Linse sowie ggf. der oder die Spiegel in einer (an sich bekannten) Kontaktglas-Fassung gehalten, die auf der dem Auge abgewandten Seite durch eine (klar durchsichtige) Staubschutzplatte bzw. Abdeckplatte abgeschlossen ist. Diese Staubschutzplatte kann erfindungsgemäß eine Reihe von zusätzlichen Funktionen wahrnehmen:

Eine derartige Abdeckplatte dient nicht nur dem Staubschutz, sondern ermöglicht auch ein Sterilisieren des erfindungsgemäßen Kontaktglases beispielsweise "in Lösung". Vor allem aber kann die Abdeckplatte auch optische Funktionen wahrnehmen:

Zur Änderung des Einstrahlwinkels kann die Abdeckplatte bspw. keilförmig ausgebildet sein.

Ferner ist es möglich durch eine schrägstehende Abdeckplatte Reflexe zu minimieren. Die Abdeckplatte sowie auch die Spiegel können ferner Träger für Markierungen zum leichteren Auffinden der Spiegelanordnung (z.B. Einkerbungen), von sog. TABO-Markierungen usw. sein.

Weiterhin können an der Abdeckplatte zusätzliche Elemente, wie Keilelemente, Linsen etc. angebracht werden. Hierbei ist es bevorzugt, daß die Abdeckplatte austauschbar ist, so daß nacheinander die unterschiedlichsten Abdeckgläser bzw. Abdeckplatten eingesetzt werden können.

Das erfindungsgemäße Kontaktglas erweitert nicht nur den (horizontalen und vertikalen) Bildwinkel vorhandener Augenuntersuchungsgeräte für den Augenhintergrund, sondern ermöglicht zusätzlich auch die Beobachtung und/oder Behandlung vorderer Augenabschnitte mit Geräten, wie beispielsweise Laser-Scanning-Ophthalmoskopen, die eigentlich nur für die Beobachtung des Augenhintergrundes ausgelegt sind:

Um z.B. die Hornhaut (Epithel, Endothel) beobachten zu können, muß die Fokusebene von der Retina zur Hornhaut verlegt werden. Dies kann mittels einer zusätzlich in den Strahlengang eingebrachten Fokussierlinse erfolgen, die bevorzugt auf die Abdeckplatte aufgesteckt wird.

Bei der Darstellung des Augenhintergrundes beispielsweise mit einem Scanning-Laser-Ophthalmoskop wird der Laserstrahl durch die Augenlinse auf den Augenhintergrund, d.h. die Retina, fokussiert. Für die Darstellung der vorderen Augenabschnitte ist damit eine zusätzliche Optik zur Fokussierung erforderlich.

Bei der Weiterbildung, bei der die augenseitige Hauptebene der zusätzlichen Linse einen Abstand von der Augenlinse hat, der gleich der Brennweite der zusätzlichen Linse ist, ist die Größe der abgetasteten Fläche und damit die Vergrößerung durch die Brennweite der zusätzlichen Linse festgelegt. Der Scan-Winkel bleibt bei dieser Anordnung konstant, der Fokusdurchmesser des abtastenden Laserstrahls verkleinert sich in gleichem Maße wie die abgetastete Strecke auf der Cornea.

Bei der dieser Anordnung bleibt jedoch beim Fokussieren auf unterschiedliche ringförmige Segmente der gekrümmten Cornea der Maßstabsfaktor nicht konstant.

Deshalb ist es besonders bevorzugt, wenn auch die auf der Seite des Untersuchungsgeräts angeordnete Hauptebene der zusätzlichen Linse einen Abstand von der Fokusebene des Untersuchungsgerätes hat, der gleich der Brennweite der zusätzlichen Linse ist, so daß die zusätzliche Linse ein telezentrisches System bildet. Betrachtet man wiederum den Fall, daß das Augenuntersuchungsgerät ein Scanning-Laser-Ophthalmoskop ist, so liegt die Scan-Pupille im Abstand der Brennweite vor der zusätzlichen Linse, so daß der scannende Strahl nach der zusätzlichen Linse einen Parallelversatz erfährt. Durch Vorfokussierung im Scanning-Laser-Ophthalmoskop wird nur die Schärfenebene, nicht jedoch der Abbildungsmaßstab verändert.

Erlaubt das Augenuntersuchungsgerät eine Umschaltung des Bildfeldes, so kann ohne Wechsel des Kontaktglases bzw. der zusätzlichen Linse zwischen einer Übersichts-Darstellung und einer Detailansicht umgeschaltet werden.

Ferner ist es möglich, einen (oder mehrere) Umlenkspiegel als Hohlfokussierspiegel auszubilden. Dies ermöglicht insbesondere eine Beobachtung des Kammerwinkels.

In jedem Falle hat diese Ausbildung den Vorteil, daß der bei der Corneamikroskopie mit hoher Vergrößerung und

4

entsprechend kleinen Brennweiten (im Bereich von 20 dpt und größer) äußerst kritische Abstand zwischen Fokussierlinse bzw. Fokussierspiegel und Cornea durch das erfindungsgemäß verwendete Kontaktglas, dessen auf das Auge aufgesetzte Linse praktisch keine sphärische Brechkraft hat, problemlos konstant gehalten wird. Andererseits wirkt sich der Abstand Fokussieroptik/Scanpupille des Scanning Laser Ophthalmoskop nicht auf die Bildschärfe, sonder nur auf den Abbildungsmaßstab aus, so daß dieser Abstand nicht so "kritisch" ist.

**Kurze Beschreibung der Zeichnung**

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

Fig. 1a      einen Längsschnitt durch ein erfindungsgemäßes Kontaktglas,

Fig. 1b      den Strahlengang bei Beobachtung über den ersten Spiegelsatz,

Fig. 1c      den Strahlengang bei Beobachtung über den zweiten Spiegelsatz,

Fig. 2      eine Variante des in Fig. 1 dargestellten Kontaktglases,

Fig. 3      eine weitere Variante des in Fig. 1 dargestellten Kontaktglases, und

Fig. 4      eine dritte Variante des in Fig. 1 dargestellten Kontaktglases.

**Darstellung von Ausführungsbeispielen**

Als Augenuntersuchungsgeräte können in Verbindung mit dem im folgenden beschriebenen Kontaktglas beliebige Untersuchungsgeräte verwendet werden, die eine Beobachtung des Augenhintergrundes bereits ohne zusätzliches Kontaktglas gestatten. Derartige Untersuchungsgeräte sind beispielsweise herkömliche Funduskameras, Ophthalmoskope oder sogenannte Scanning-Laserophthal-moskope, wie sie beispielsweise aus der EP-A-0 145 563, den japanischen Patentveröffentlichungen 61-5730 und 50-138822, der deutschen Patentschrift 32 45 939 oder der WO 88/03396 bekannt sind. Deshalb soll im folgenden auf die Ausbildung der Untersuchungsgerät nicht näher eingegangen werden; in verschiedenen Figuren ist jedoch der Strahlengang des Untersuchungsgeräts "nach der letzten optischen Fläche" des Geräts dargestellt.

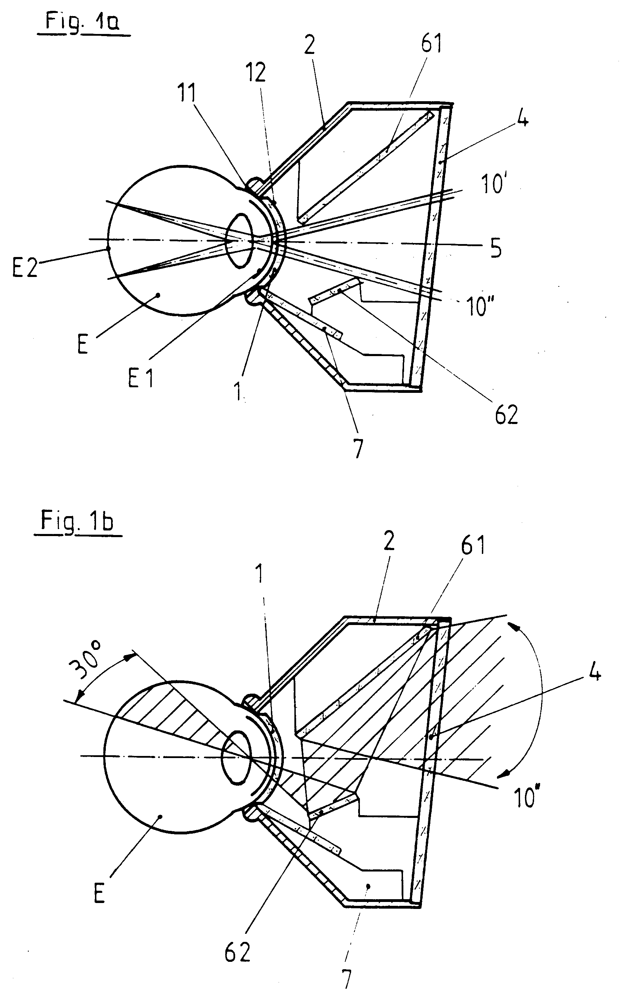

Fig. 1 zeigt einen Längsschnitt durch ein erfindungsgemäßes Kontaktglas, das eine Linse 1 aufweist, deren augenseitige Fläche 11 derart gekrümmt ist, daß das Glas auf die Cornea E1 eines zu untersuchenden Auges E aufgesetzt werden kann. Die augenseitige Fläche kann dabei eine sphärische, aber auch eine asphärische Fläche sein, deren Asphärizität insbesondere so gewählt ist, daß die augenseitige Fläche 11 dem Corneaverlauf folgt.

Die zweite Fläche 12 der Linse 1 ist so gewählt, daß die Linse 1 im Bereich der optischen Achse eine sphärische Wirkung von praktisch 0 dpt hat, d.h. die Linse 1 hat keine optische Wirkung für durchtretende Strahlen. Gegebenenfalls kann diese Linse jedoch eine astigmatische Wirkung zum Ausgleich eines Augenastigmatismus haben.

Zum Ausgleich chromatischer Fehler des Auges ist es jedoch bevorzugt, wenn die Fläche 12 eine asphärische Fläche ist, deren Asphärizität so gewählt ist, daß sie die zum Rand hin abweichende Brechkraft des menschlichen Auges ausgleicht. Beschreibt man die Flächenschnitte der in der Regel rotationssymmetrischen asphärischen Fläche durch die in der Optik übliche "Asphärengleichung", so ist die Pfeilhöhe z der asphärischen Vorderfläche, d.h. der Abstand in Richtung der optischen Achse eines Flächenpunktes vom Scheitelpunkt der Fläche durch die folgende Gleichung gegeben:

$$z = Cr^2/(1 + (1-(K+1)C^2 r^2)^{-1/2}) + C_4 \cdot r^4 + C_6 \cdot r^6 + ...$$

hierbei bedeuten:

r:      Abstand des Flächenpunktes von der optischen Achse,
C:      Krümmung der Fläche im Scheitel: $C = 1/R = D_1/(n-1)$
R:      Krümmungsradius der Fläche
K:      Kegelschnittkoeffizient
$C_i$:      Asphärenkoeffizienten (i=4,6,...)
$D_1$:      Flächenbrechkraft im Scheitel,

n:      Brechungsindex des Linsenmaterials

Das dargestellte Ausführungsbeispiel kann beispielsweise folgende Werte haben:

Radius der augenseitigen Fläche 11: 8,31 mm

Radius der asphärischen Fläche 12 im Scheitel:

$$R = 8,6 \text{ mm}$$
$$K = -0,594$$
$$C_4 = 2*10^{-6}$$
$$C_6 = 1,4*10^{-6}$$

Die Linsendicke auf der optischen Achse beträgt 2,0 mm und der Brechungsindex des Glasmaterials 1,5.

Gehalten ist die Linse 1 in einer Linsenfassung 2, die ergonomisch griffgünstig für die Untersuchungsperson gestaltet ist, und die an ihrem vorderen, d.h. dem Auge E entgegengesetzten und dem einer nicht dargestellten Untersuchungsperson zugewandten Ende von einer (durchsichtigen) Staubschutzplatte bzw. einer Abdeckplatte 4 abgeschlossen ist. Die Abdeckplatte 4 ist nicht senkrecht zur optischen Achse 5 des zu untersuchenden Auges E und der Linse 1 angeordnet. Diese Anordnung minimiert Reflexe.

Ferner sind in der Linsenfassung 2 Spiegel 61 und 62 sowie 7 angeordnet, die bei dem gezeigten Ausführungsbeispiel einfache Planspiegel sind. Der Spiegel 7 ist in dem dargestellten Längsschnitt der um 90° gegenüber seiner tatsächlichen Anordnung verdreht eingezeichnet.

Im folgenden soll die Funktionsweise des in Fig. 1 dargestellten Kontaktglases unter Bezugnahme auf die Teilfiguren a bis c näher beschrieben werden. Dabei soll ohne Beschränkung des allgemeinen Erfindungsgedankens davon ausgegangen werden, daß das Kontaktglas mit einem Scanning-Laserophthalmoskop (SLO) mit einem Bildwinkel von 30° verwendet wird, also einem Gerät, bei dem ein Laser-Beleuchtungsstrahl 10 eine abtastende Bewegung auf dem Augenhintergrund ausführt, wobei die Scanelemente optisch konjugiert zur Augenpupille ist.

Bei der in Teilfigur 1a dargestellten "zentralen Stellung" des Scanning-Laserophthalmoskops gehen die beiden in einer "Scanebene auftretenden Randstrahlen" 10' und 10" des Beleuchtungslichtes zwischen der Spiegelanordnung 61, 62 und 7 hindurch. Da die Linse 1 keine optische Wirkung hat, wird der Laserstrahl 10 in gleicher Weise wie ohne auf das Auge E aufgesetztes Kontaktglas auf dem Augenhintergrund E2 fokussiert.

Entsprechend wird auch ein Behandlungs-Laserstrahl auf eine zu koagulierende Stelle des Augenhintergrundes fokussiert.

Schwenkt man das Scanning-Laserophthalmoskop in der Zeichenebene, so treffen die Strahlen 10 nunmehr auf den Spiegel 61 auf, werden von diesem auf den Spiegel 62 umgelenkt, und von dem Spiegel 62 durch die Linse 1 auf einen sich an den zentralen Bereich anschließenden Bereich des Augenhintergrundes E2 abgebildet. Wiederum gilt das Entsprechende für den Strahl eines Behandlungslasers.

Bei einer weiteren Schwenkung des Scanning-Laserophthalmoskops trifft der Laserstrahl von auf den Spiegel 7 auf, und wird von diesem durch die Linse 1 auf einen sehr peripheren Bereich des Augenhintergrundes, d.h. auf einen seitlichen Bereich abgelenkt (Fig. 1c).

In den Figuren 1b und 1c sind exemplarisch die Beobachtung bzw. Behandlung des Augenhintergrundes in "horizontalen peripheren Bereichen" dargestellt. Selbstverständlich kann auch der "vertikale" Beobachtungsbereich des Geräts zur Untersuchung des Augenhintergrundes dadurch erweitert werden, daß das Kontaktglas bei einer Schwenkung des Untersuchungsgeräts um eine vertikale Achse zusätzlich um die "Augenachse", d.h. die optische Achse des Auges gedreht wird.

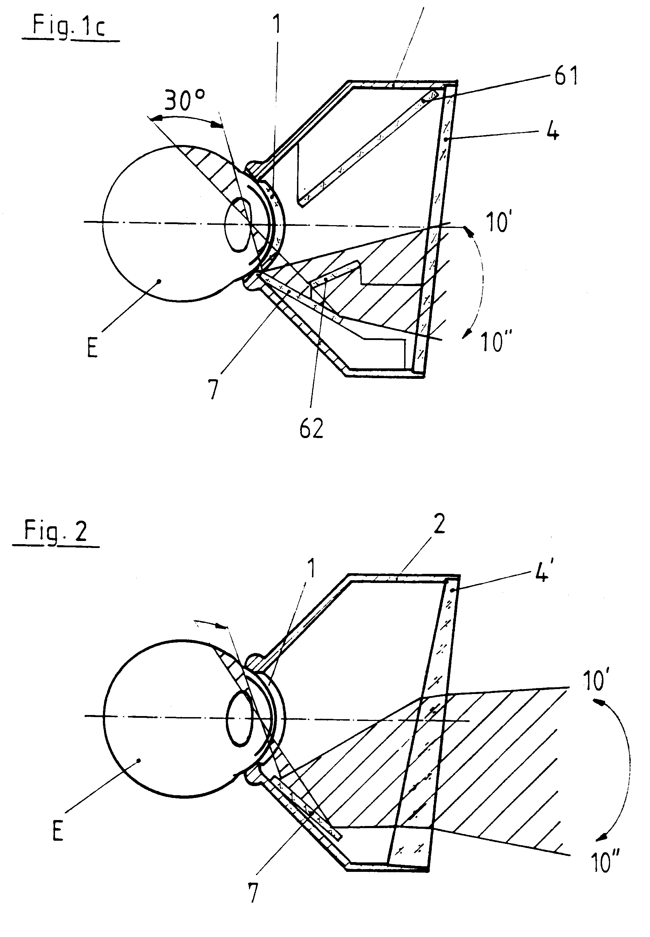

In den Figuren 2 bis 4 sind Varianten des in Fig. 1 dargestellten Kontaktglases gezeigt. Dabei bezeichnen gleiche Bezugszeichen jeweils gleiche Elemente wie in Fig. 1, so daß auf eine erneute Vorstellung verzichtet wird:

Fig. 2 zeigt eine Variante, bei dem eine extreme Fundusperipherie-Beobachtung durch eine keilförmige Ausbildung der Abdeckplatte 4', die damit als Ablenkprisma wirkt, möglich wird.

Das erfindungsgemäß vorgesehene Kontaktglas erweitert nicht nur den (horizontalen und vertikalen) Bildwinkel vorhandener Augenuntersuchungsgerate für den Augenhintergrund, sondern ermöglicht zusätzlich auch die Beobachtung und/oder Behandlung vorderer Augenabschnitte mit derartigen Geräten:

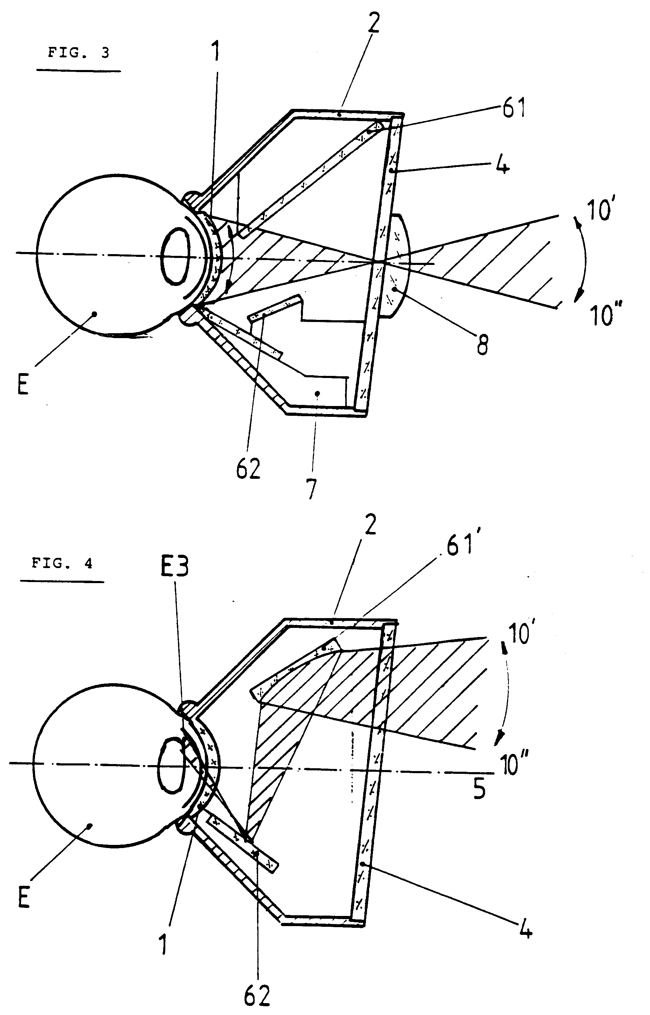

Um z.B. zentral die Hornhaut (Epithel, Endothel) beobachten zu können, muß die Fokusebene von der Retina zur Hornhaut verlegt werden. Dies kann gemäß Fig. 3 durch eine zusätzlich mittig in den Strahlengang eingebrachten Fokussierlinse 8 erfolgen, die bevorzugt auf die Abdeckplatte 4 aufgesteckt bzw. durch Auswechslen der Abdeckplatte eingebracht wird.

Dabei ist es besonders bevorzugt, wenn die zusätzliche Linse 8 ein telezentrisches System bildet. Betrachtet man wiederum den Fall, daß das Augenuntersuchungsgerät ein Scanning-Laser-Ophthalmoskop ist, so liegt die Scan-Pupille im Abstand der Brennweite vor der zusätzlichen Linse, so daß der scannende Strahl nach der zusätzlichen Linse einen Parallelversatz erfährt. Durch Vorfokussierung im Scanning-Laser-Ophthalmoskop daher wird nur die Schärfenebene, nicht jedoch der Abbildungsmaßstab verändert.

Ferner ist es gemäß Fig. 4 möglich, zur Beobachtung des Kammerwinkels beispielsweise den Umlenkspiegel 61' als Hohlfokussier-spiegel mit typischerweise ca 20 dpt auszubilden, um die Fokussierwirkung der nicht durchsetzten Augenlinse E3 auszugleichen. Diese Ausbildung hat gegenüber einer Linsenanordnung gemäß Fig. 3 den Vorteil der Reflexfreiheit und ermöglicht insbesondere eine Beobachtung des Kammerwinkels, ohne daß die zentrale Beobachtung des Augenhintergrundes beeinflußt würde.

Vorstehend ist die Erfindung anhand von Ausführungsbeispielen ohne Beschränkung des allgemeinen Erfindungsgedankens beschrieben worden, innerhalb dessen selbstverständlich die verschiedensten Modifikationen möglich sind:

So ist es möglich, mehr als zwei Spiegelsätze vorzusehen. Ferner können die einzelnen Elemente vergütet oder mit einer speziellen Laser-Beschichtung versehen werden. Weiterhin kann in das Bild-aufnehmende System ein Signal eingegeben werden, das die Stellung des Kontaktglases relativ zum Beobachtungsstrahlengang angibt. Auch können Spiegel beweglich ausgeführt werden. Ferner ist es möglich, die Abdeckplatte austauschbar und/oder mit Zusatzelementen - wie zusätzlichen Fokussierlinsen, Keilprismen etc - bestückbar auszubilden. Selbstverständlich ist es auch möglich, spezielle Markierungen etc vorzusehen. Letztlich kann die augenseitige Fläche 11 der Linse 1 auch in Anpassung an die Geometrie der Cornea asphärisch ausgebildet werden.

**Patentansprüche**

1.  Vorrichtung zur Untersuchung und/oder zur Behandlung des Auges (E), mit

    - einem Untersuchungsgerät und
    - einem Kontaktglas, das eine Linse (1) aufweist, deren augenseitige Fläche (11) zum Aufsetzen auf das Auge dem Verlauf der Cornea angepaßt ist,

    **gekennzeichnet** durch die Kombination folgender Merkmale:
    das Untersuchungsgerät ist ein Gerät, das eine unmittelbare Beobachtung des Augenhintergrundes ohne Zwischenbild erlaubt, und die Linse (1) hat zumindest im Bereich der optischen Achse (5) eine sphärische Wirkung D, für die gilt:

    $$- 0,5 \, dpt < D < 0,5 \, dpt.$$

2.  Vorrichtung nach Anspruch 1,
    dadurch **gekennzeichnet**, daß an der dem Auge gegenüberliegenden Seite des Kontaktglases ein optisches Element (4) vorgesehen ist, welches eine durchsichtige Abdeckplatte mit ebenen Flächen ist.

3.  Vorrichtung nach Anspruch 2,
    dadurch **gekennzeichnet**, daß zwischen der Linse (1) und dem optischen Element wenigstens ein Spiegel (61, 62, 7) vorgesehen ist, der die Beobachtung und/oder Behandlung peripherer Augenabschnitte ermöglicht.

4.  Vorrichtung nach Anspruch 3,
    dadurch **gekennzeichnet**, daß der Spiegel außerhalb des Beobachtungs- und/oder Behandlungs-Strahlengangs für die zentralen Bereiche angeordnet ist.

5.  Vorrichtung nach Anspruch 3 oder 4,
    dadurch **gekennzeichnet**, daß der Spiegel beweglich ist.

6.  Vorrichtung nach einem der Ansprüche 3 bis 5,
    dadurch **gekennzeichnet**, daß zwei Sätze von Spiegeln vorgesehen sind, die die Beobachtung und/oder Behandlung unterschiedlicher peripherer Bereiche ermöglichen.

7.  Vorrichtung nach einem der Ansprüche 3 bis 6,
    dadurch **gekennzeichnet**, daß sich der zentrale Beobachtungs- und Behandlungsbereich und der bzw. die über Spiegel beobachtbare Bereiche ohne Überschneidung aneinander anschließen.

8.  Vorrichtung nach einem der Ansprüche 3 bis 7,
    dadurch **gekennzeichnet**, daß wenigstens ein Teil der Spiegel (61') eine optische Wirkung aufweist.

9.  Vorrichtung nach Anspruch 8,

dadurch **gekennzeichnet**, daß die optische Wirkung der Spiegel Abbildungsfehler in den peripheren Augenbereichen wenigstens teilweise kompensiert.

10. Vorrichtung nach einem der Ansprüche 2 bis 9,
dadurch **gekennzeichnet**, daß die auf das Auge aufsetzbare Linse (1) sowie gegebenenfalls der oder die Spiegel (61, 62, 7) in einer Kontaktglas-Fassung (2) gehalten sind, die auf der dem Auge (E) abgewandten Seite durch das optische Element (4) abgeschlossen ist.

11. Vorrichtung nach Anspruch 10,
dadurch **gekennzeichnet**, daß das optische Element (4') zur Änderung des Einstrahlwinkels keilförmig ausgebildet ist.

12. Vorrichtung nach Anspruch 9,
dadurch **gekennzeichnet**, daß das optische Element (4) schräg zur optischen Achse (5) der auf das Auge (E) aufsetzbaren Linse (1) angeordnet ist.

13. Vorrichtung nach einem der Ansprüche 1 bis 12,
dadurch **gekennzeichnet**, daß zur Beobachtung und/oder Behandlung vorderer Augenabschnitte eine zusätzliche Linse (8) in den Strahlengang einbringbar ist.

14. Vorrichtung nach Anspruch 13 in Verbindung mit Anspruch 2,
dadurch **gekennzeichnet**, daß die zusätzliche Linse (8) auf dem optischen Element (4) angebracht ist.

15. Vorrichtung nach einem der Ansprüche 2 bis 14,
dadurch **gekennzeichnet**, daß das optische Element (4) austauschbar ist.

16. Kontaktglas nach einem der Ansprüche 2 bis 15,
dadurch **gekennzeichnet**, daß auf dem optischen Element (4) und/oder dem oder den Spiegeln Markierungen und insbesondere TABO-Markierungen vorgesehen sind.

## Claims

1. A device for examining and/or treating the eye (E), with

   - an examining apparatus and
   - a contact glass, which has lens (1) whose surface (11) on the eye side is matched to the shape of the cornea for placing on the eye,

   characterized by the combination of the following features:
   the examining apparatus is an apparatus which allows direct observation of the fundus of the eye without an intermediate image, and the lens (1) has a spherical power D at least in the region of the optical axis (5) for which there applies:

   $$-0.5\,dpt < D < 0.5\,dpt.$$

2. A device according to claim 1, characterized in that an optical element (4) is provided on the side of the contact glass opposite the eye and is a transparent cover plate with flat surfaces.

3. A device according to claim 2, characterized in that at least one mirror (61, 62, 7) is provided between the lens (1) and the optical element and enables observation and/or treatment of peripheral eye sections.

4. A device according to claim 3, characterized in that the mirror is arranged outside the observation and/or treatment beam path for the central regions.

5. A device according to claim 3 or 4, characterized in that the mirror is movable.

6. A device according to any of claims 3 to 5, characterized in that two sets of mirrors are provided, which enable observation and/or treatment of different peripheral regions.

**7.** A device according to any of claims 3 to 6, characterized in that the central observation and/or treatment region and the region or regions observable through mirrors adjoin each other without intersecting.

**8.** A device according to any of claims 3 to 7, characterized in that at least a part of the mirror (61') has optical power.

**9.** A device according to claim 8, characterized in that the optical power of the mirror compensates at least partially for optical aberrations in the peripheral eye regions.

**10.** A device according to any of claims 2 to 9, characterized in that the lens (1) which can be applied to the eye as well as optionally the mirror or mirrors (61, 62, 7) are held in a contact glass holder (2) which is closed by the optical element (4) on the side remote from the eye (E).

**11.** A device according to claim 10, characterized in that the optical element (4') is of wedge form so as to alter the angle of incidence.

**12.** A device according to claim 9, characterized in that the optical element (4) is arranged obliquely with respect to the optical axis (5) of the lens (1) which can be applied to the eye (E).

**13.** A device according to any of claims 1 to 12, characterized in that an additional lens (8) can be introduced into the beam path for observation and/or treatment of front eye sections.

**14.** A device according to claim 13 in conjunction with claim 2, characterized in that the additional lens (8) is fitted on the optical element (4).

**15.** A device according to any of claims 2 to 14, characterized in that the optical element (4) is interchangeable.

**16.** A contact glass according to any of claims 2 to 15, characterized in that markings, in particular TABO markings, are made on the optical element (4) and/or the mirror or mirrors.

## Revendications

**1.** Dispositif ou appareil pour l'examen et/ou le traitement de l'oeil (E), comportant

- un appareil d'examen
- et un verre de contact qui présente une lentille (1) dont la surface (11) située du côté de l'oeil est adaptée à la forme de la cornée pour la mise en place sur l'oeil,

caractérisé par la combinaison des caractéristiques suivantes:
l'appareil d'examen est un appareil qui permet l'observation directe du fond de l'oeil sans image intermédiaire, et la lentille (1) a, au moins dans la zone de l'axe optique (5), une puissance sphérique (D), à laquelle s'applique la relation:

$$-0,5 \text{ dioptrie} < D < 0,5 \text{ dioptrie}$$

**2.** Dispositif selon la revendication 1,
caractérisé en ce que, du côté du verre de contact opposé à l'oeil, est prévu un élément optique (4) qui est une plaque de couverture transparente comportant des surfaces planes.

**3.** Dispositif selon la revendication 2,
caractérisé en ce qu'entre la lentille (1) et l'élément optique est prévu au moins un miroir (61, 62, 7) qui permet l'observation et/ou le traitement de parties périphériques de l'oeil.

**4.** Dispositif selon la revendication 3,
caractérisé en ce que le miroir est disposé en dehors du trajet des rayons en vue de l'observation et/ou du traitement des zones centrales.

**5.** Dispositif selon la revendication 3 ou 4,
caractérisé en ce que le miroir peut être déplacé.

6. Dispositif selon l'une des revendications 3 à 5,
   caractérisé en ce que deux jeux de miroirs sont prévus, lesquels permettent l'observation et/ou le traitement de zones périphériques différentes.

7. Dispositif selon l'une des revendications 3 à 6,
   caractérisé en ce que la zone centrale d'observation et de traitement et la, ou les, zone, ou zones susceptible(s) d'être observée(s) au moyen du miroir se raccordent sans chevauchement.

8. Dispositif selon l'une des revendications 3 à 7,
   caractérisé en ce qu'au moins une partie des miroirs (61') est dotée d'un effet optique.

9. Dispositif selon la revendication 8,
   caractérisé en ce que l'effet optique des miroirs compense au moins partiellement les défauts ou imperfections d'image (aberrations) dans les zones périphériques de l'oeil.

10. Dispositif selon l'une des revendications 2 à 9,
    caractérisé en ce que la lentille (1) susceptible d'être mise en place sur l'oeil, ainsi que le ou les miroirs (61, 62, 7) sont maintenus dans une monture (2) de verre de contact qui, du côté opposé à l'oeil (E), est fermée par l'élément optique (4).

11. Dispositif selon la revendication 10,
    caractérisé en ce que l'élément optique (4') a une forme de coin en vue de modifier l'angle d'incidence.

12. Dispositif selon la revendication 9,
    caractérisé en ce que l'élément optique (4) est disposé obliquement par rapport à l'axe optique (5) de la lentille (1) susceptible d'être mise en place sur l'oeil (E).

13. Dispositif selon l'une des revendications 1 à 12,
    caractérisé en ce qu'il est possible de mettre en place une lentille supplémentaire dans le trajet des rayons en vue de l'examen et/ou du traitement des parties antérieures de l'oeil.

14. Dispositif selon la revendication 3, en liaison avec la revendication 2,
    caractérisé en ce que la lentille supplémentaire (8) est mise en place sur l'élément optique (4).

15. Dispositif selon l'une des revendications 2 à 14,
    caractérisé en ce que l'élément optique (4) est interchangeable.

16. verre de contact selon l'une des revendications 2 à 15,
    caractérisé en ce que, sur l'élément optique (4) et/ou sur les miroirs sont prévues des marques et en particulier des marques TABO.

Fig. 1a

Fig. 1b

Fig. 1c

Fig. 2

FIG. 3

FIG. 4